# EUROPÄISCHE PATENTANMELDUNG

(11) **EP 4 403 123 A1**
(43) Veröffentlichungstag der Anmeldung: **24.07.2024**
(21) Anmeldenummer: 23152010.7
(22) Anmeldetag: 17.01.2023
(51) Int. Cl.: A61B 18/04, A61B 18/00

(54) **PLASMASONDE ZUR MEDIZINISCHEN BEHANDLUNG VON GEWEBE**

(71) Anmelder: Erbe Elektromedizin GmbH, 72072 Tübingen (DE)
(72) Erfinder: HEYM, Johannes, 72074 Tuebingen (DE); WALZ, Martin, 72108 Rottenburg (DE)
(74) Vertreter: Rüger Abel Patentanwälte PartGmbB

(57) **Zusammenfassung**

Eine flexible Plasmasonde weist ein Kopfstück (12) mit wenigstens einem Plasmaaustrittsfenster (25) auf, das weder radial, noch axial, sondern in dem sich verjüngenden Endbereich (18) des Kopfstücks mit schräg zur Längsmittelachse (L) orientierter Öffnungsrichtung (0) angeordnet ist. Eine derartige Plasmasonde (10) erleichtert und verbessert die Behandlung von biologischem Gewebe, insbesondere Läsionen (11) in Körperlumina aufgrund der erreichbaren größeren Nähe zwischen der Elektrode (20) und der Läsion (11).

## Beschreibung

Die Erfindung betrifft eine Plasmasonde zur Behandlung von biologischem Gewebe, insbesondere für den endoskopischen Einsatz.

Plasmasonden, insbesondere Argon-Plasmasonden, werden häufig zur Gewebekoagulation oder Ablation genutzt. Verschiedene Bauformen solcher Plasmasonden können zum Beispiel aus der JP 2002-301088 entnommen werden. Demnach sind Plasmasonden mit axialen und/oder seitlichen Plasmaaustrittsöffnungen bekannt. Insbesondere ist es bekannt, solche Plasmasonden aus einem flexiblen Schlauch mit einem distal daran befestigten Kopfstück auszubilden, das eine mit HF-Spannung versorgte Elektrode enthält. Das Kopfstück kann an seinem distalen Ende gerundet sein und sowohl seitliche, als auch eine axiale Plasmaaustrittsöffnung aufweisen.

Die GB 2 573 128 A offenbart eine Plasmasonde mit einem flexiblen Schlauch und einem an seinem distalen Ende sphärisch gerundeten Endstück. Im zylindrischen Abschnitt des Endstücks sind seitliche Plasmaaustrittsöffnungen vorgesehen.

Die US 2013/0090644 A1 offenbart eine Plasmasonde mit einer als Sieb ausgebildeten halbkugelförmig gerundeten Endkappe. Ein weiteres Instrument offenbart die WO 2011/055368 A2.

Um die Plasmaaustrittsöffnung möglichst nahe an zu behandelndes Gewebe heranzubringen, offenbart die EP 1 397 082 B1 eine Plasmasonde mit zylindrischer Grundstruktur und zwei seitlichen Plasmaaustrittsöffnungen, die jeweils einer Schraubenlinie folgen. Im Bereich dieser Plasmaaustrittsöffnungen ist der Sondenkörper abknickbar, so dass beim Aufsetzen des distalen Endes der Sonde auf das Gewebe und entsprechend seitlicher Kraftausübung auf die Sonde bei den Plasmaaustrittsöffnungen eine Knickstelle entsteht, an denen die Plasmaaustrittsöffnung dem Gewebe näher kommt, als bei gestreckter Form der Sonde. Dies ist insbesondere bei der Behandlung von krankhaften Gewebeneubildungen vorteilhaft, die über sonstiges Gewebe hervorstehen, wie beispielsweise Darmpolypen oder anderen Läsionen. Jedoch setzt dieses Prinzip eine Flexibilität der Sonde im Bereich der Plasmaaustrittsöffnung voraus, was eine konstruktive Herausforderung sein kann.

Davon ausgehend ist es Aufgabe der Erfindung, eine Plasmasonde vereinfachter Bauart zu schaffen, die ein nahes Heranbringen des Plasmaaustrittsfensters an zu behandelndes Gewebe gestattet.

Diese Aufgabe wird mit der Plasmasonde nach Anspruch 1 gelöst:
Die erfindungsgemäße Plasmasonde weist einen flexiblen Schlauch auf, der an seinem distalen Ende mit einem starren Kopfstück versehen ist, das einen sich in distaler Richtung verjüngenden Endbereich aufweist. Das Kopfstück weist ein oder mehrere Plasmaaustrittsfenster auf, die sich wenigstens teilweise in den sich verjüngenden Endbereich des Kopfstücks hinein erstrecken. In das Kopfstück hinein erstreckt sich auch eine Elektrode, die über einen elektrischen Leiter mit einem HF-Generator verbindbar und von diesem mit geeigneter HF-Spannung versorgbar ist. Von der Elektrode ionisiertes durch den Schlauch nachströmendes Gas bildet einen Plasmastrom, der seitlich zur Längsrichtung der Plasmasonde aus dem Plasmaaustrittsfenster quer zur Elektrode austritt. Weil sich das Plasmaaustrittsfenster in dem sich verjüngenden Endbereich des Endstücks befindet oder sich zumindest in diesen hinein erstreckt, können Gewebeläsionen zielgerichtet behandelt werden. Der Abstand zwischen der Elektrode und dem zu behandelndem Gewebe kann sehr gering gehalten werden und zugleich ist der Zielort der Behandlung sehr genau festlegbar. Vorzugsweise beginnt die Verjüngung des Kopfstücks an dem proximalen Ende des Plasmaaustrittsfensters oder der Plasmaaustrittsfenster.

Die Plasmasonde ist vorzugsweise monopolar ausgebildet, das heißt, die Plasmasonde weist nur eine einzige Elektrode auf und der Strom fließt von der Elektrode zu dem zu behandelnden Gewebe und von diesem über eine am Patienten angebrachte Neutralelektrode zu dem Generator zurück. Weil der Abstand zwischen der Elektrode und dem Gewebe durch das erfindungsgemäße Design minimiert ist, kann nicht nur der Einwirkungsort des Plasmastrahls genau festgelegt werden, sondern es kann auch mit relativ geringer Leistung gearbeitet und auf das Gewebe eingewirkt werden, um keine über das notwendige Maß hinausgehende Gewebeschädigung zu verursachen.

Die erfindungsgemäße Plasmasonde eignet sich insbesondere für den Einsatz in engen Hohlgefäßen, bei denen die Sonde beispielsweise über ein Endoskop im wesentlichen parallel zum Verlauf eines Körperlumens in demselben geführt und nur das Ende des Endeskops und der Plasmasonde etwas auf die zu behandelnde Stelle hin abgewinkelt wird. Das gerundete Kopfstück ermöglicht dabei, dass das distale Ende der Sonde auf dem zu behandelnden Gewebe gleitet und wirkt somit wie eine Kufe. Dies gilt insbesondere wenn der sich verjüngende Endbereich nicht nur halbkugelförmig gerundet, sondern darüber hinaus etwas länglich gerundet ausgebildet ist. Vorzugsweise weist das Endstück im Längsschnitt eine gerundete, eckenlose Kontur auf. Er kann die Form eines abgestumpften gerundeten Kegels aufweisen, das hießt eines Kegels mit gerundeter Spitze oder auch gerundeten Flanken, so dass seine Mantellinien bogenförmig verlaufen. Gerade mit dieser Form wird eine besonders gute Wirkung erhalten.

Vorzugsweise weist die Plasmasonde ausschließlich seitliche Plasmaaustrittsfenster mit quer, dabei aber etwas schräg zur Längsrichtung der Plasmasonde orientierter Plasmaaustrittsrichtung auf. Vorzugsweise ist die erfindungsgemäße Plasmasonde dabei am distalen Ende geschlossen. Das heißt, es ist keine Plasmaaustrittsöffnung mit axialer Austrittsrichtung vorgesehen. Zwischen den Plasmaaustrittsfenstern sind trennende in Axialrichtung verlaufende Stege angeordnet. In Umgangsrichtung gemessen ist die Summe der Breiten der Plasmaaustrittsfenster größer, vorzugsweise erheblich größer, als die Summe der Breiten der zwischen den Plasmaaustrittsfenstern vorhandenen Stege.

Vorzugsweise weist das Kopfstück an gleicher Axialposition mehrere in Umfangsrichtung voneinander beabstandete Plasmaaustrittsfenster auf, die ausnahmslos wenigstens teilweise in dem sich verjüngenden Endbereich angeordnet sind. Weiter vorzugsweise ist in dem Kopfstück somit lediglich ein einziger Kranz von Plasmaaustrittsfenstern vorgesehen, das heißt, alle Plasmaaustrittsfenster sind an der gleichen Axialposition angeordnet.

Die Elektrode kann in dem Kopfstück fixiert und dazu beispielsweise am distalen Ende des Kopfstücks in diesem verankert sein. Bei einer anderen, gegenwärtig bevorzugten Ausführungsform ist das Ende der Elektrode jedoch frei, so dass kein direkter thermischer Kontakt zwischen der Elektrode und dem Isoliermaterial des Kopfstücks gegeben ist. Dadurch kann die Temperatur des Kopfstücks außen niedrig gehalten werden, um ein Ankleben am Gewebe zu vermeiden.

Die Elektrode kann über einen geeigneten Halter, beispielsweise ein das Schlauchlumen durchquerendes Blechstück in dem Schlauch oder in einem rohrförmigen Fortsatz des Kopfstücks oder auch sowohl im Schlauch, als auch im Kopfstück gehalten sein. Dadurch wird die Elektrode in der Nähe des distalen Endes der Plasmasonde zentriert, so dass die Plasmasonde nach allen seitlichen Richtungen gleichermaßen arbeiten kann.

Der Schlauch weist zumindest ein gasführendes Lumen auf, das sich von seinem proximalen Ende bis zu dem Kopfstück erstreckt. Über ein geeignetes am proximalen Ende des Schlauchs angebrachtes Verbindungsmittel ist das Lumen mit einer Gasquelle verbindbar und somit mit einem geeigneten Gas, vorzugsweise Argon längs durchströmbar. Der Schlauch kann alternativ mehrere gasführende Lumen aufweisen, die sich von dem proximalen Ende zu dem distalen Ende des Schlauchs erstrecken und gemeinsam in das Kopfstück münden.

Weitere Einzelheiten vorteilhafter Ausführungsformen der Erfindung sind Gegenstand von Ansprüchen, sowie Gegenstand von Zeichnung und der zugehörigen Beschreibung. In der Zeichnungen zeigen:
Figur 1 ein Endoskop mit einer erfindungsgemäßen Sonde, angeschlossen an speisende Geräte in schematisierter Darstellung,
Figur 2 das distale Ende des Endoskops und der Sonde beim Einsatz an behandlungsbedürftigem biologischem Gewebe,
Figur 3 das distale Ende der erfindungsgemäßen Sonde in einer Seitenansicht,
Figur 4 die Sonde nach Figur 3 geschnitten in Längsrichtung,
Figur 5 die Sonde nach Figur 3 und 4 geschnitten entlang der Linie V-V in Figur 4,
Figur 6 das Kopfstück der Sonde nach Figur 3 und 4 in längsgeschnittener Darstellung,
Figur 7 die Sonde nach Figur 2, 3 und 4 im Einsatz,
Figur 8 eine abgewandelte Ausführungsform der erfindungsgemäßen Sonde in schematisierter Seitenansicht,
Figur 9 eine abgewandelte Ausführungsform eines Schlauchs einer Sonde in quer geschnittener Darstellung,
Figur 10 eine asymmetrische erfindungsgemäße Sonde mit lediglich einem Plasmaaustrittsfenster in schematisierter Seitenansicht und
Figur 11 eine erfindungsgemäße Plasmasonde mit rotationssymmetrischem Kopfstück und einem einzigen seitlichen Plasmaaustrittsfenster in Seitenansicht.

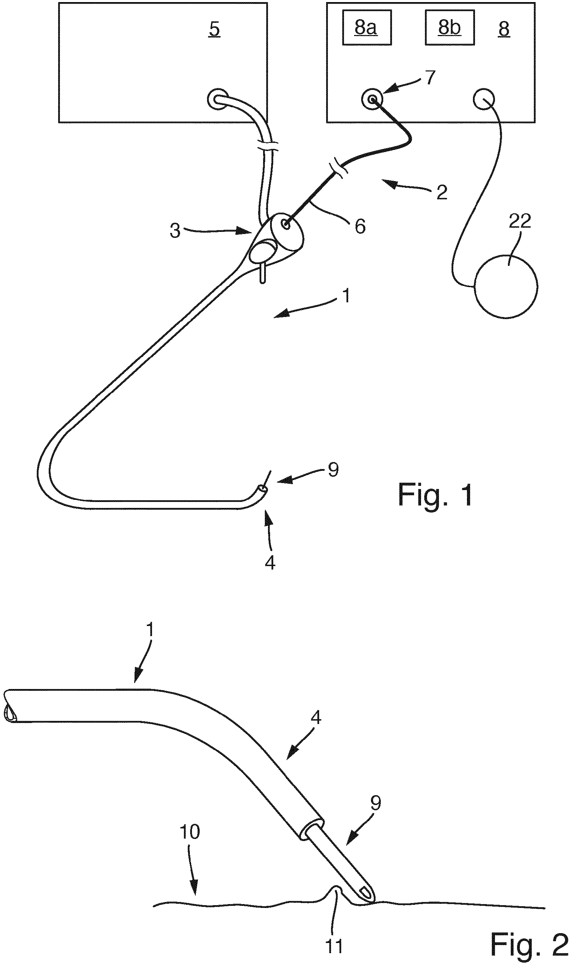

Figur 1 veranschaulicht eine Einrichtung zur Plasmabehandlung von Gewebe, insbesondere von Oberflächen in Körperlumen mit einem Katheter 1 und einer in dessen Arbeitskanal angeordneten Plasmasonde 2. Der Katheter 1 weist an seinem proximalen Ende einen Steuerkopf 3 auf, mit dem sich eine Abwinkelbewegung seines distalen Endes 4 steuern lässt. Außerdem kann der Katheter 3 an ein Gerät 5 angeschlossen sein, das zum Beispiel zur Wiedergabe eines an dem distalen Ende 4 aufgenommenen Bildes und/oder zur Versorgung des Katheters 1 mit geeigneten Betriebsmedien, beispielsweise Gasen, Spülfluiden oder dergleichen eingerichtet ist.

Die Plasmasonde 2 umfasst einen Schlauch 6, dessen proximales Ende über ein geeignetes Verbindungsmittel 7 an ein Gerät 8 anschließbar und angeschlossen ist, dass die Plasmasonde 2 mit Strom und Gas eingerichtet ist. Das Gerät enthält eine Gasquelle 8a zur Abgabe von Gas, z.B. Inertgas, und einen Generator zur Erzeugung von chirurgischem Strom.

Wie Figur 1 und insbesondere auch Figur 2 erkennen lässt, weist die Plasmasonde ein distales Ende 9 auf, das in Längsrichtung aus dem Arbeitskanal des Katheters 1 herausschiebbar ist, um eine Gewebeoberfläche 10 und insbesondere daran vorhandene behandlungsbedürfte Stellen 11, sogenannte Läsionen einer Behandlung zu unterziehen. Solche behandlungsbedürftigen Stellen können benigne insbesondere aber auch maligne Wucherungen oder deren Vorstufen sein, beispielsweise sogenannten Polypen im Dickdarm oder anderen Hohlorganen.

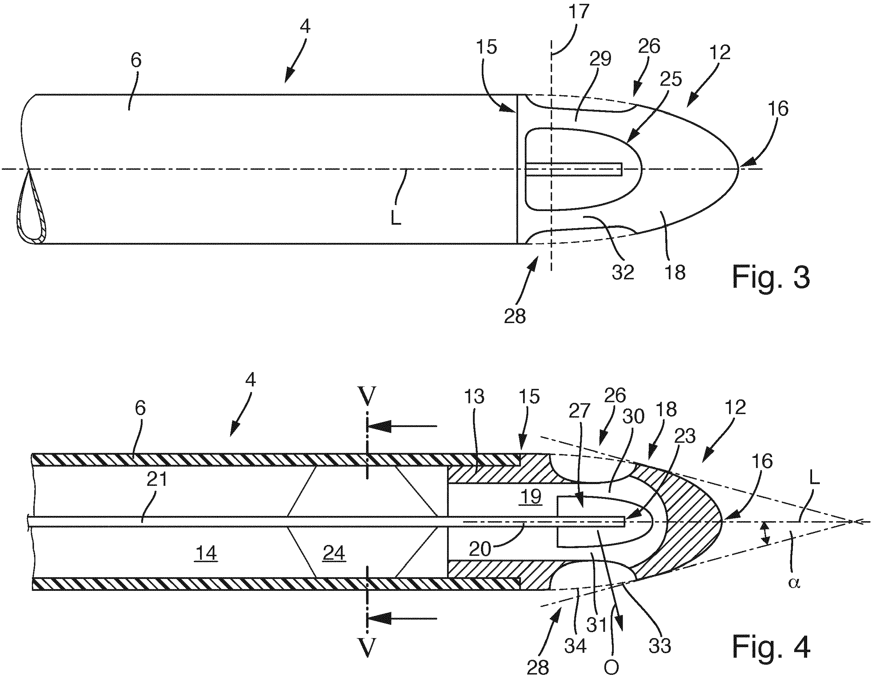

Das distale Ende 9 der Plasmasonde 2 ist in den Figuren 3 und 4 zur Verdeutlichung des besonderen Aufbaus der Plasmasonde 2 gesondert veranschaulicht. Wie ersichtlich ist in das offene distale Ende des vorzugsweise aus Kunststoff bestehenden flexiblen Schlauchs 6 ein Kopfstück 12 eingesetzt, das mit einem rohrförmigen Fortsatz 13 (Figur 4) in das sich durch die gesamte Länge längs durch den Schlauch 6 erstreckende Lumen 14 ragt. In diesem Ausführungsbeispiel ist der Schlauch 6 ein einlumiger Schlauch ohne innere Unterteilung.

Das Kopfstück 12 ist vorzugsweise aus einem elektrisch isolierenden, wärmebeständigen Material, wie beispielsweise Keramik oder einem sehr temperaturstabilen Kunststoff ausgebildet. Es schließt bei einer Fuge 15 vorzugsweise glatt, das heißt stufenlos an die Außenseite des Schlauchs 6 an und verjüngt sich bis zu der gerundeten distalen Spitze 16 des Kopfstücks 12. Die Verjüngung kann schon an der Fuge 15 beginnen oder auch, wie es Figur 3 veranschaulicht, erst in einer gewissen distalen Distanz, zum Beispiel an einer in Figur 3 gestrichelt eingetragenen Linie 17. Zwischen der Spitze 16 und der gestrichelten Linie 17 weist das Kopfstück 12 einen sich in distaler Richtung verjüngenden Endbereich 18 auf, der die Form eines gerundeten Kegels haben kann. Der Radius und der Durchmesser des Endbereichs 18 nimmt in distaler Richtung bis zu der distalen Spitze 16 hin kontinuierlich, d.h. vorzugsweise stufenlos, ab. Sie sich von der Spitze 16 in Richtung der Fuge 15 durch den Endbereich 18 erstreckenden Mantellinie kann zum Beispiel elliptisch, parabolisch oder anderweitig gekrümmt sein.

Das Endstück 12 ist an seiner Spitze 16 geschlossen, ansonsten aber hohl ausgebildet und umschließt einen Innenraum 19 (Figur 4), der mit dem Lumen 14 kommuniziert und in den eine Elektrode 20 ragt.

Die Elektrode 20 wird durch einen Metallstift, beispielweise aus Edelstahl gebildet und kann mit einer Beschichtung versehen sein, beispielsweise aus einem Metall, dessen Schmelzpunkt niedriger ist als der des Edelstahls und das wenig oxidationsanfällig ist, wie beispielweise Silber. Die Elektrode 20 ist über eine Leitung 21 mit dem Gerät 8 verbunden, das zur Strombeaufschlagung der Elektrode 20 einen Generator aufweist. Die Elektrode 20 kann auch durch das distale, nicht isolierte Ende der Leitung 21 selbst gebildet sein. Der Gegenpol zu der Elektrode 20 ist an eine Neutralelektrode 22 angeschlossen (siehe Figur 1), die zur Behandlung des Patienten an einer geeigneten Stelle desselben großflächig anzubringen ist.

Die Elektrode 20 ist vorzugsweise zentrisch in dem Kopfstück 12 gehalten und erstreckt sich längs und koaxial zu einer Längsmittelachse L der Plasmasonde 2. Die Elektrode 20 kann mit ihrer Spitze 23 frei in dem Innenraum 19 schwebend gehalten sein. Alternativ kann die Spitze 23 in eine dazu entsprechend vorgesehene, zentral auf der in Längsrichtung L verlaufenden Mittelachse angeordnete, in Figur 4 nicht veranschaulichte Tasche greifend angeordnet sein, um in jedem Fall zentrisch gehalten zu bleiben.

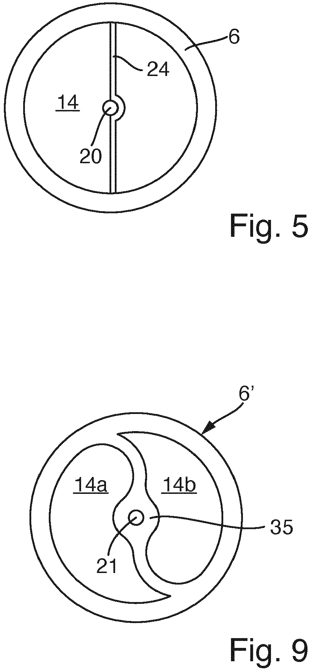

Zusätzlich oder alternativ kann wie in Figur 4 dargestellt, ein Elektrodenhalter 24 vorgesehen sein, der als Blechteil ausgebildet sein kann. Figur 5 veranschaulicht die Anordnung des Elektrodenhalters 24 in dem Lumen 14 des Schlauchs 6.

Das Kopfstück 12 weist mindestens ein, vorzugsweise mehrere Plasmaaustrittsfenster 25, 26, 27, 28 auf, die, wie insbesondere aus Figur 3 hervorgeht, sich insbesondere in den sich verjüngenden Endbereich 18 hinein erstrecken oder deren proximales Ende mit dem proximalen Ende des Endbereichs 18 zusammenfällt. Für alle Ausführungsformen gilt, dass sich die Elektrode 20 vorzugsweise in den sich verjüngenden Endbereich 18 des Kopfstücks 12 hinein erstreckt, wobei die widerum mehr als die halbe axiale Längserstreckung des Plasmaaustrittsfensters 25 einnimmt. Dadurch wird das Plasma an der Position der Plasmaaustrittsfenster erzeugt und somit wenig Wärme auf das Kopfstück 12 übertragen. Die Plasmasonde 2 eignet sich deswegen insbesondere zur Erzeugung und Applikation von weitgehend thermischem Plasma auf Gewebe. Die Plasmatemperatur kann oberhalb 40°C, 60°C oder 100°C oder noch weiter darüber liegen. Die Plasmasonde ist somit nicht nur zur Kaltplasmaapplikation sondern auch zur Applikation von warmem oder heißem Plasma geeignet.

Die Plasmaaustrittsfenster 25 bis 28 sind untereinander vorzugsweise gleich ausgebildet und bezüglich der Längsrichtung L an gleicher Stelle angeordnet. Insofern hat das Kopfstück 12 eine Drehsymmetrie, indem es bei der vierfenstrigen Ausführung durch eine Drehung um 90° um die in Längsrichtung L verlaufende Mittelachse jeweils wieder in Deckung mit der vorigen Position überführbar ist. Bei zweifenstrigen Sonden ist eine 180°-Drehsymmetrie vorhanden; bei dreifenstrigen Sonden eine 120°-Drehsymmetrie.

Zwischen den Fenstern 25 bis 28 sind Stege 29 bis 32 ausgebildet (siehe Figuren 3 und 4), die in Längsrichtung L gemessen vorzugsweise eine Länge aufweisen, die größer ist als ihre in Umfangsrichtung gemessene Breite. Außerdem ist die Breite jedes Stegs 29 bis 32 vorzugsweise geringer als die in Umfangsrichtung zu messende Breite jedes Plasmaaustrittsfensters 25 bis 28. Die Form jedes Plasmaaustrittsfensters 25 bis 28, kann gerundet sein. Z.B. kann die Form durch ein Polygon, z.B. Dreieck oder Trapez, mit gerundeten Ecken und/oder gerundeten Kanten bestimmt sein.

Durch die wenigstens teilweise Anordnung der Plasmaaustrittsfenster 25 bis 28 in dem sich verjüngenden Endbereich 18 des Kopfstücks 12 sind die Plasmaaustrittsfenster 25 bis 28 schräg zu der in Längsrichtung orientierten Mittelachse L orientiert. Ihre von der proximalen Begrenzung zur distalen Begrenzung verlaufende gedachte Linie 33 (Figur 4) schließt mit der Mittelachse einen spitzen Winkel α ein. Entsprechend ist die quer zu der Längsrichtung L verlaufende Öffnungsrichtung O des Plasmaustrittsfensters 25 - 28 in einem Winkel zu der Längsrichtung L geneigt, der kleiner als 90° ist.

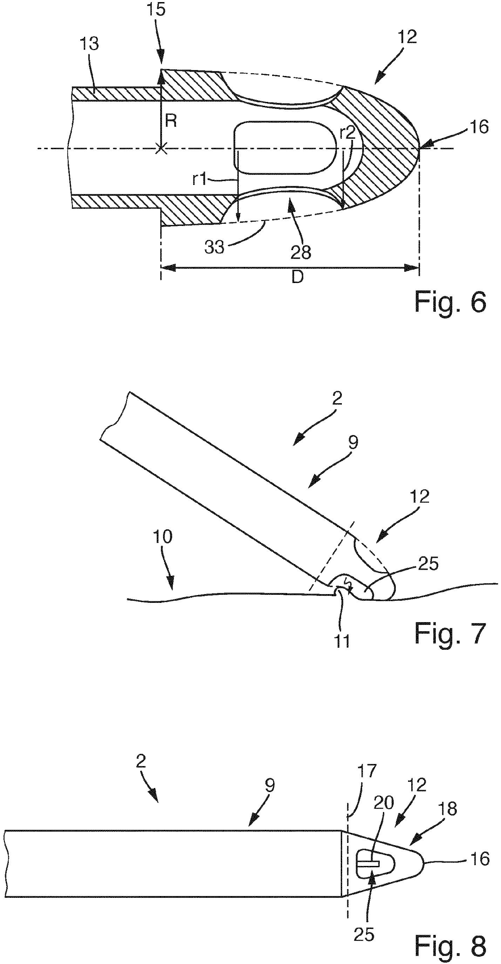

Auch die in Figur 4 gestrichelt eingetragene Kontur 34 des Endbereichs verringert ihren Radius im Verlauf jeder Öffnung 25 bis 28. Dies ist insbesondere aus Figur 6 entnehmbar, in der der Radius r1 des Plasmaaustrittsfensters 28 und der Radius r2 am distalen Rand des Plasmaaustrittsfensters 28 eingetragen sind.

Aus der gleichen Figur ist erkennbar, dass das längliche Kopfstück 12 zumindest vorzugsweise eine von der Fuge 15 zu seiner Spitze 16 zu messende Länge D aufweist, die größer ist als sein Radius R, insbesondere größer als sein größter im Querschnitt zu messender Radius. Das Kopfstück 12 erhält dadurch gute Gleiteigenschaften, so dass sich die Plasmasonde 2 auch in enge Körperlumen gut und leicht einschieben lässt.

Die insoweit beschriebene Plasmasonde 2 arbeitet mit Verweis auf Figur 7 wie folgt:
In Betrieb ist das Lumen 14 von der Gasquelle 8a mit einem geeigneten Gas, beispielsweise einem Inertgas, insbesondere Argon beaufschlagt, so dass sich entlang des Leiters 21 eine Gasströmung ausbildet, die aus den Plasmaaustrittsfenstern 25 bis 28 entweicht. Die Elektrode 20 wird von dem Generator 8b mit HF-Spannung versorgt, so dass das Gas ionisiert wird und sich ein Plasmastrom zu dem biologischen Gewebe 10 hin ausbildet. Im Idealfall hat der Plasmastrom eine Strömungsrichtung, die weitgehend mit der Öffnungsrichtung (Figur 4) übereinstimmt.

In dem Ausführungsbeispiel nach Figur 7 ist das Plasmaaustrittsfenster 25 dem Gewebe 10 und dem behandlungsbedürftigen Gewebebereich 11 zu gekehrt. Aufgrund der Nähe des behandlungsbedürftigen Bereichs 11 und der hinter dem Plasmaaustrittsfenster 25 stehenden Elektrode ergibt sich nun ein intensiver Plasmastrom, der insbesondere und vorwiegend die behandlungsbedürftige Stelle 11 trifft. Zugleich kann die Plasmasonde 2 mit ihrem Kopfstück 12 sehr leicht auf dem Gewebe 10 entlang gleitend geführt werden. Dabei kann die Plasmasonde 2 in einem flachen (d.h. spitzen) Winkel zu der Oberfläche des Gewebes 10 geführt werden und so alle Läsionen erreichen. Auch ist die Plasmasonde 2 für enge Lumina geeignet, in die sie insbesondere wegen des Fehlens einer axialen Gasaustrittsöffnung gut und leicht einschiebbar ist. Aufgrund der geringen Distanz zwischen der Elektrode 20 und dem Gewebe 10 in Folge der Schrägstellung der Plasmaausstrittsfenster 25 bis 28 kann mit geringen Plasmaleistungen gearbeitet und somit schonend behandelt werden.

An der insoweit beschriebenen Plasmasonde 2 sind eine Vielzahl von Variationen möglich. Figur 9 veranschaulicht eine hinsichtlich der Ausbildung des Schlauchs 6 abgewandelte Ausführungsform mit einem Schlauch 6`. Dieser weist zwei oder mehrere Lumen 14a, 14b auf, die durch Trennwände voneinander abgeteilt sind. Die Trennwände erstrecken sich von einem mittleren Abschnitt 35 zu der Wandung des Schlauchs 6' und gehen dabei ununterbrochen von dem proximalen Ende 7 zu dem distalen Ende des Schlauchs 6 durch. Der zentrale Bereich 35 kann den elektrischen Leiter 21 enthalten und zugleich als Elektrodenhalter dienen. Im Übrigen gilt die vorige Beschreibung der Ausführungsform gemäß Figur 1 bis 7, entsprechend mit der Maßgabe, dass die beiden die Teillumina 14a, 14b voneinander trennenden Wände im Bereich des Fortsatzes 13 des Kopfstücks 12 entfernt sind. Ein solcher Schlauch ist insbesondere für einge Biegeradien geeignet. Die Gefahr des Abknicken des Lumens und der Hemmung des Gassflusses dadurch ist wesentlich gemindert. In den einzelnen Teillumina 14a, 14b geführte eventuell unterschiedliche Gasflüsse werden in dem Kopfstück 12 zusammengeführt. Der Innenraum 19, insbesondere der in dem Fortsatz 13 vorhandene Teil des Innenraums 19 kann insoweit eine Gassammel- und Ausgleichskammer bilden.

Figur 8 veranschaulicht eine Ausführungsform einer Plasmasonde 2 mit einem Kopfstück 12, dessen Endbereich 18 als gerader Kegel mit gerundeter Spitze 16 ausgebildet ist. Die Plasmaaustrittsfenster 25 (sowie weitere, zum Beispiel 26 bis 28) können als Bogendreieckförmige Fenster ausgebildet sein. Außerdem können sie vollständig in dem sich verjüngenden Endbereich 18 angeordnet sein.

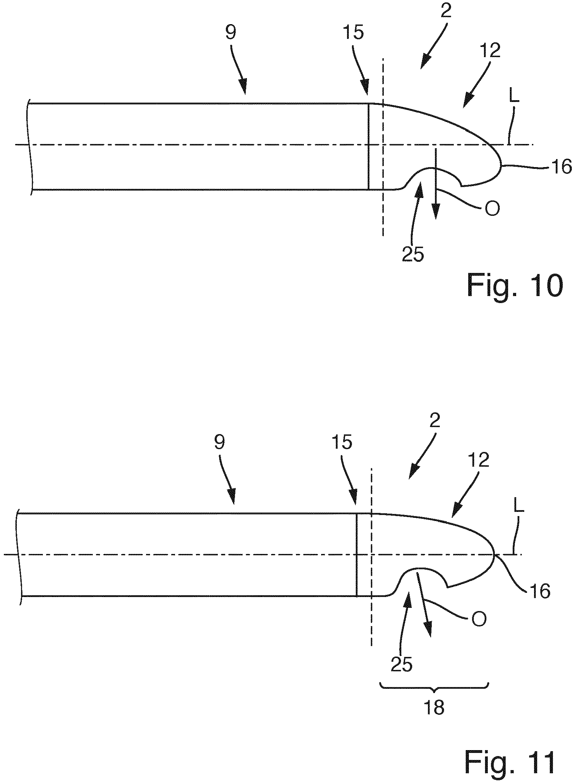

Figur 10 veranschaulicht eine weiter abgewandelte Plasmasonde 2 mit asymmetrischem Kopfstück 12. Dieses weist lediglich ein einziges Plasmaaustrittsfenster 25 auf. Seine distale Spitze 16 liegt außerhalb der Längsmittelachse L. Das Plasmaaustrittsfenster 25 ist in dem sich verjüngenden Endbereich 18 angeordnet, wobei seine Öffnungsrichtung O wie in Figur 10 dargestellt in einem rechten Winkel zu der Längsmittelachse L oder wie bei den anderen Ausführungsformen in einem spitzen Winkel zu der Längsmittelachse L orientiert sein kann.

Figur 11 veranschaulicht wiederum eine Ausführungsform einer Plasmasonde 2 mit einem Kopfstück 12, dessen distale Spitze 16 zentrisch zu der Längsmittelachse L angeordnet ist. Die zu den Figuren 1 bis 9 gegebenen Ausführungen gelten für die Ausführungsform nach Figur 11 entsprechend, jedoch mit der Maßgabe, dass lediglich ein einziges Plasmaaustrittsfenster 25 vorgesehen ist. Die Öffnungsrichtung O ist, wie schon ausführlichem Zusammenhang mit Figur 4 dargelegt, in einem spitzen Winkel zu der Längsmittelachse L orientiert. Die Plasmaaustrittsöffnung 25 kann teilweise oder wie dargestellt vollständig in dem sich verjüngenden Endbereich 18 angeordnet sein.

Eine flexible Plasmasonde weist ein Kopfstück 12 mit wenigstens einem Plasmaaustrittsfenster 25 auf, das weder radial, noch axial, sondern in dem sich verjüngenden Endbereich 18 des Kopfstücks mit schräg zur Längsmittelachse L orientierter Öffnungsrichtung O angeordnet ist. Eine derartige Plasmasonde 10 erleichtert und verbessert die Behandlung von biologischem Gewebe, insbesondere Läsionen 11 in Körperlumina aufgrund der erreichbaren größeren Nähe zwischen der Elektrode 20 und der Läsion 11.

### Bezugszeichen:

- 1: Katheter
- 2: Plasmasonde
- 3: Steuerkopf
- 4: distales Ende des Katheters 1
- 5: Gerät
- 6: Schlauch (6` in Figur 9)
- 7: Verbindungsmittel am proximalen Schlauchende
- 8: Gerät zum Betrieb der Plasmasonde 2
- 8a: Gasquelle in dem Gerät 8
- 8b: Generator in dem Gerät 8
- 9: distales Ende der Plasmasonde 2
- 10: Gewebeoberfläche
- 11: behandlungsbedürftige Stelle
- 12: Kopfstück
- 13: rohrförmiger Fortsatz des Kopfstücks
- 14: Lumen des Schlauchs 6 (14a, 14b in Figur 9)
- 15: Fuge
- 16: Spitze
- 17: gestrichelte Linie in Figur 3
- 18: Endbereich
- 19: Innenraum
- 20: Elektrode
- 21: Leitung
- 22: Neutralelektrode
- L: Längsrichtung und Längsmittelachse der Plasmasonde 2
- 23: Spitze der Elektrode 20
- 24: Elektrodenhalter
- 25 - 28: Plasmaaustrittsfenster
- 29 - 32: Stege zwischen den Plasmaaustrittsfenstern
- 33: gedachte Linie
- α: spitzer Winkel
- 34: gestrichelte Linie / Kontur
- 35: Mittelabschnitt des Schlauchs 6'
- O: Öffnungsrichtung des Plasmaaustrittsfensters 25

## Patentansprüche

1. Plasmasonde (2) zur Behandlung von biologischem Gewebe (10, 11), insbesondere für den endoskopischen Einsatz,
mit einem flexiblen Schlauch (6), der sich von seinem proximalen Ende entlang einer Längsmittelachse (L) bis zu seinem distalen Ende (9) erstreckt, wobei das proximale Ende (7) des Schlauchs (6) an eine Gasquelle (8a) anschließbares Lumen (14) aufweist,
mit einem in dem Schlauch (6) angeordneten elektischen Leiter (21), der sich von dem proximalen Ende (7) bis zu dem distalen Ende (9) erstreckt und der an einen elektrischen Generator (8b) anschließbar ist,
mit einer Elektrode (20), die mit dem elektrischen Leiter (21) verbunden ist, und
mit einem Kopfstück (12), das an dem distalen Ende (9) des Schlauchs (6) befestigt ist und einen sich in distaler Richtung verjüngenden Endbereich (18) aufweist,
wobei in dem Kopfstück (12) wenigstens ein Plasmaaustrittsfenster (25) ausgebildet ist,
**dadurch gekennzeichnet,**
**dass** das Plasmaaustrittsfenster (25) wenigstens teilweise in dem sich verjüngenden Endbereich (18) angeordnet ist.

2. Plasmasonde nach Anspruch 1, **dadurch gekennzeichnet, dass** das Kopfstück (12) aus einem hitzebeständigen, elektrisch isolierenden Material ausgebildet ist.

3. Plasmasonde nach einem der vorstehenden Ansprüche,
dadurch gekennzeichent, dass das Plasmaaustrittsfenster (25) quer zu der Längsmittelachse (L) orientiert ist.

4. Plasmasonde nach einem der vorstehenden Ansprüche, dadurch gekennzeichent, dass das Kopfstück (12) an gleicher Axialposition mehrere in Umfangsrichtung voneinander beabstandete Plasmaaustrittsfenster (25 - 28) aufweist, die ausnahmslos wenigstens teilweise in dem sich verjüngenden Endbereich (18) angeordnet sind.

5. Plasmasonde nach einem der vorstehenden Ansprüche, **dadurch gekennzeichnet, dass** der sich verjüngende Endbereich (18) gerundet ist.

6. Plasmasonde nach einem der vorstehenden Ansprüche, dadurch gekennzeichent, dass der sich verjüngende Endbereich (18) länglich gerundet ist.

7. Plasmasonde nach einem der vorstehenden Ansprüche, dadurch gekennzeichent, dass das Kopfstück (12) an seinem schlauchseitigen Ende einen Kreisquerschnitt mit einem Radius (R) aufweist, der kleiner ist, als die von dem schlauchseitigen Ende (15) zu dem von dem Schlauch (6) weg liegenden Ende (16) zu messende Länge (D).

8. Plasmasonde nach einem der vorstehenden Ansprüche, dadurch gekennzeichent, dass die Elektrode (20) in dem Schlauch (6) fixiert ist.

9. Plasmasonde nach einem der vorstehenden Ansprüche, dadurch gekennzeichent, dass die Elektrode (20) in dem Kopfstück (12) fixiert ist.

10. Plasmasonde nach einem der vorstehenden Ansprüche, dadurch gekennzeichent, dass die Elektrode (20) ein distales Ende (23) aufweist, das das Kopfstück (12) nicht berührt.

11. Plasmasonde nach einem der vorstehenden Ansprüche, dadurch gekennzeichent, dass der Schlauch (6) ein einziges gasführendes Lumen (14) aufweist.

12. Plasmasonde nach einem der vorstehenden Ansprüche, dadurch gekennzeichent, dass der Schlauch (6) mehrere gasführende Lumen (14a, 14b) aufweist.

13. Plasmasonde nach einem der vorstehenden Ansprüche, dadurch gekennzeichent, dass das Kopfstück (12) nur ein einziges Plasmaaustrittsfenster (25) aufweist.

14. Plasmasonde nach einem der vorstehenden Ansprüche, dadurch gekennzeichent, dass der sich verjüngende Bereich (18) des Kopfstücks (12) zu der Längsmittelachse (L) rotationssymmetrisch ausgebildet ist.

15. Plasmasonde nach einem der vorstehenden Ansprüche, dadurch gekennzeichent, dass der sich verjüngende Bereich (18) des Kopfstücks (12) zu der Längsmittelachse (L) asymmetrisch ausgebildet ist.
